# EUROPEAN PATENT SPECIFICATION

(11) **EP 2 632 458 B1**
(45) Date of publication and mention of the grant of the patent: **12.08.2015**
(21) Application number: 11779606.0
(22) Date of filing: 25.10.2011
(51) Int. Cl.: A61K 31/4709, A61K 31/505, A61K 31/506, A61K 31/519, A61P 27/02

(54) **DOSING REGIMES FOR THE TREATMENT OF OCULAR VASCULAR DISEASE**
DOSIERSCHEMATA UND ZUR BEHANDLUNG VON OKULAREN GEFÄSSERKRANKUNGEN
SCHÉMAS POSOLOGIQUES POUR LE TRAITEMENT DES MALADIES VASCULAIRES OCULAIRES

(30) Priority: 27.10.2010 US 407218 P
(43) Date of publication of application: 04.09.2013
(73) Proprietor: Novartis AG, 4056 Basel (CH)
(72) Inventor: BRIGELL, Mitchell, Cambridge, Massachusetts 02139 (US); END, Peter, CH-4002 Basel (CH); HOSAGRAHARA, Vinayak, Mumbai 400071 (IN); JAFFEE, Bruce, Cambridge, Massachusetts 02139 (US); MEREDITH, Erik, Cambridge, Massachusetts 02139 (US); NEWTON, Ronald, Cambridge, Massachusetts 02139 (US); POOR, Stephen, Cambridge, Massachusetts 02139 (US); QIU, Yubin, Cambridge, Massachusetts 02139 (US)
(74) Representative: von Sprecher, Georg
(86) International application number: PCT/EP2011/068682
(87) International publication number: WO 2012/055884

(56) References cited:
- US-A1- 2010 111 963
- TAKAHASHI KYOICHI ET AL: "Suppression and regression of choroidal neovascularization by the multitargeted kinase inhibitor pazopanib.", ARCHIVES OF OPHTHALMOLOGY APR 2009 LNKD- PUBMED:19365030, vol. 127, no. 4, April 2009 (2009-04), pages 494-499, XP002665908, ISSN: 1538-3601
- CHUNG E J ET AL: "Inhibition of choroidal neovascularisation in mice by systemic administration of the multikinase inhibitor, sorafenib.", THE BRITISH JOURNAL OF OPHTHALMOLOGY JUL 2009 LNKD- PUBMED:19028740, vol. 93, no. 7, July 2009 (2009-07), pages 958-963, XP009154932, ISSN: 1468-2079
- WIEGAND S J ET AL: "VEGF trap both prevents experimental choroidal neovascularization and causes regression of established lesions in non-human primates", IOVS, vol. 46, no. Suppl. S, 2005, page 1411, XP009154915, & ANNUAL MEETING OF THE ASSOCIATION-FOR-RESEARCH-IN-VISION-AND-OPH THALM OLOGY; FT LAUDERDALE, FL, USA; MAY 01 -05, 2005 ISSN: 0146-0404
- BENJAMIN P NICHOLSON ET AL: "A review of clinical trials of anti-VEGF agents for diabetic retinopathy", GRAEFE'S ARCHIVE FOR CLINICAL AND EXPERIMENTAL OPHTHALMOLOGY ; INCORPORATING GERMAN JOURNAL OF OPHTHALMOLOGY, SPRINGER, BERLIN, DE, vol. 248, no. 7, 20 February 2010 (2010-02-20), pages 915-930, XP019847969, ISSN: 1435-702X
- ESKENS F A L M ET AL: "The clinical toxicity profile of vascular endothelial growth factor (VEGF) and vascular endothelial growth factor receptor (VEGFR) targeting angiogenesis inhibitors; A review", EUROPEAN JOURNAL OF CANCER, PERGAMON PRESS, OXFORD, GB, vol. 42, no. 18, 1 December 2006 (2006-12-01), pages 3127-3139, XP025104562, ISSN: 0959-8049, DOI: 10.1016/J.EJCA.2006.09.015 [retrieved on 2006-12-01]
- BASHSHUR Z F ET AL: "Intravitreal Bevacizumab for the Management of Choroidal Neovascularization in Age-related Macular Degeneration", AMERICAN JOURNAL OF OPHTHALMOLOGY, OPHTHALMIC PUBL, CHICAGO, IL, US, vol. 142, no. 1, 1 July 2006 (2006-07-01), pages 1-9, XP025046250, ISSN: 0002-9394, DOI: 10.1016/J.AJO.2006.02.037 [retrieved on 2006-07-01]
- KOURLAS H ET AL: "Pegaptanib sodium for the treatment of neovascular age-related macular degeneration: A review", CLINICAL THERAPEUTICS, EXCERPTA MEDICA, PRINCETON, NJ, US, vol. 28, no. 1, 1 January 2006 (2006-01-01), pages 36-44, XP025059736, ISSN: 0149-2918, DOI: 10.1016/J.CLINTHERA.2006.01.009 [retrieved on 2006-01-01]

## Description

### INTRODUCTION

The invention relates to the use of Vascular Endothelial Growth Factor Receptor 2 (VEGF-R2) inhibitors in the treatment of ocular disease. More particularly, the invention relates to the use of intermittent dosing of certain VEGF-R2 inhibitors in the treatment of ocular vascular diseases. In certain dosing regimes provided herein, the VEGF-R2 inhibitor is administered once every 5 days or less frequently, once every 5 to 21 days or once every 6, 7, or 8 days.

### BRIEF DESCRIPTION OF THE ART

VEGF-R2 has been recognized as a target for the treatment of various proliferative and neovascular diseases including certain oncology and ophthamology indications. In particular, sorafenib (Bayer, 4-[4-[[4-chloro-3-(trifluoromethyl)phenyl]carbamoylamino]phenoxy]-*N-*methyl-pyridine-2-carboxamide), pazopanib (Glaxo Smith-Kline, 5-[[4-[(2,3-Dimethyl-2H-indazol-6-yl)methylamino]-2-pyrimidinyl]amino]-2-methylbenzolsulfonamide), and tivozanib (Aveo Pharmaceuticals, 1-(2-chloro-4-((6,7-dimethoxyquinolin-4-yl)oxy)phenyl)-3-(5-methylisoxazol-3-yl)urea) are registered or are in advanced clinical develoment for the treatment of various oncology indications.

Oral sorafenib has been administered to three patients with neovascular adult macular degeneration (AMD) alone or in combination with intravitreal bevacizumab on a daily or thrice weekly administration with uncertain benefit because of the low number of patients treated and the fact that the disease may wane without therapy in occasional patients. See: T. Diago, et. al. Mayo Clin. Proc. 2008;83*2):231-234 and M. Kernt, et. al., Acta Ophthamologica, 2008(86) 456-458.

The use of VEGF-R2 inhibitors for treatment of ophthalmic diseases has been challenged by systemic toxicity concerns and the extended periods of administration necessary to treat ophthalmic vascular diseases.

### BRIEF SUMMARY OF THE INVENTION

The invention provides intermittent dosing regimens and the use in the treatment or prevention of ocular vascular disease using said dosing regimens in which a VEGF-R2 inhibitior selected from the group consisting of 5-[[4-[(2,3-Dimethyl-2H-indazol-6-yl)methylamino]-2 pyrimidinyl]amino]-2- methylbenzolsulfonamide, 5-((S)-6-Methyl-5,6,7,8-tetrahydro-pyrido[3,4-d]pyrimidin-4-yloxy)-indole-1-carboxylic acid [5-(1-methyl-cyclopropyl)-2H-pyrazol-3-yl]-amide; 6-(6-Amino-pyrimidin-4-yloxy)-naphthalene-1-carboxylic acid (3-trifluoromethyl-phenyl)-amide; (-)-5-((S)-7-Acetyl-6-methyl-5,6,7,8-tetrahydro-pyrido[3,4-d]pyrimidin-4-yloxy)-indole-1- carboxylic acid (5-cyclopropyl-isoxazol-3-yl)-amide; 5-(5,6,7,8-Tetrahydro-pyrido[3,4-d]pyrimidin-4-yloxy)-indole-1-carboxylic acid [5-(1-methyl-cyclopropyl)-2H-pyrazol-3-yl]-amide; 1-(2-chloro-4-((6,7-dimethoxyquinolin-4-yl)oxy)phenyl)-3-(5-methylisoxazol-3-yl)urea; and 6-(6-Hydroxymethyl-pyrimidin-4-yloxy)-naphthalene-1-carboxylic acid (3-trifluoromethyl- phenyl)-amide, or a pharmaceutically acceptable salt thereof, is administered once every five days or less frequently. Preferred dosing regimens include those in which the VEGF-R2 inhibitor is dosed once every 5 to 21 days, once every 5 to 14 days, once every 6 to 10 days, once every 6, 7, or 8 days or once every 7 days. The intermittent dosing regimens of the invention provide efficacy comparable to daily administration of the same dose of said VEGF-R2 inhibitor while also providing reduced systemic safety liabilities compared to daily administration. More particularly, the dosing regimens provided by the invention exhibit a sustained concentration of said VEGF-R2 inhibitor in ocular tissue over the time period between sequential administration of said VEGF-R2 inhibitor and exhibit rapid clearance of said VEGF-R2 inhibitor from the plasma of the patient. The combination of infrequent dosing, rapid clearance from the plasma and sustained intraocular exposure enhances the safety of the administered drugs while providing substantial efficacy.

In certain aspects, the invention provides a vascular endothelial growth factor receptor 2 (VEGF-R2) inhibitor selected from the group consisting of 5-[[4-[(2,3-Dimethyl-2H-indazol-6-yl)methylamino]-2 pyrimidinyl]amino]-2- methylbenzolsulfonamide, 5-((S)-6-Methyl-5,6,7,8-tetrahydro-pyrido[3,4-d]pyrimidin-4-yloxy)-indole-1-carboxylic acid [5-(1-methyl-cyclopropyl)-2H-pyrazol-3-yl]-amide; 6-(6-Amino-pyrimidin-4-yloxy)-naphthalene-1-carboxylic acid (3-trifluoromethyl-phenyl)-amide; (-)-5-((S)-7-Acetyl-6-methyl-5,6,7,8-tetrahydro-pyrido[3,4-d]pyrimidin-4-yloxy)-indole-1- carboxylic acid (5-cyclopropyl-isoxazol-3-yl)-amide; 5-(5,6,7,8-Tetrahydro-pyrido[3,4-d]pyrimidin-4-yloxy)-indole-1-carboxylic acid [5-(1-methyl-cyclopropyl)-2H-pyrazol-3-yl]-amide; 1-(2-chloro-4-((6,7-dimethoxyquinolin-4-yl)oxy)phenyl)-3-(5-methylisoxazol-3-yl)urea; and 6-(6-Hydroxymethyl-pyrimidin-4-yloxy)-naphthalene-1-carboxylic acid (3-trifluoromethyl- phenyl)-amide, or a pharmaceutically acceptable salt thereof, for use in the treatment of ocular vascular diseases wherein the dosing frequency is once every 5 days or less frequently, or more preferably is dosed once every 5 to 21 days. With the reduced frequency of dosing, the systemic exposure of the compound, as measured by plasma concentration, is reduced whilst maintaining an elevated ocular concentration.

Certain preferred VEGF-R2 inhibitors selected from the group consisting of 5-[[4-[(2,3-Dimethyl-2H-indazol-6-yl)methylamino]-2pyrimidinyl]amino]-2- methylbenzolsulfonamide, 5-((S)-6-Methyl-5,6,7,8-tetrahydro-pyrido[3,4-d]pyrimidin-4-yloxy)-indole-1-carboxylic acid [5-(1-methyl-cyclopropyl)-2H-pyrazol-3-yl]-amide; 6-(6-Amino-pyrimidin-4-yloxy)-naphthalene-1-carboxylic acid (3-trifluoromethyl-phenyl)-amide; (-)-5-((S)-7-Acetyl-6-methyl-5,6,7,8-tetrahydro-pyrido[3,4-d]pyrimidin-4-yloxy)-indole-1- carboxylic acid (5-cyclopropyl-isoxazol-3-yl)-amide; 5-(5,6,7,8-Tetrahydro-pyrido[3,4-d]pyrimidin-4-yloxy)-indole-1-carboxylic acid [5-(1-methyl-cyclopropyl)-2H-pyrazol-3-yl]-amide; 1-(2-chloro-4-((6,7-dimethoxyquinolin-4-yl)oxy)phenyl)-3-(5-methylisoxazol-3-yl)urea; and 6-(6-Hydroxymethyl-pyrimidin-4-yloxy)-naphthalene-1-carboxylic acid (3-trifluoromethyl- phenyl)-amide, or a pharmaceutically acceptable salt thereof, that are suitable for use in the treatment provided herein include those VEGF-R2 inhibitors which provide efficacy when administered once every week. Compounds which provide elevated ocular exposure and rapid plasma clearance are suitable for use according to the invention.

In one aspect, the invention provides uses of VEGR-R2 inhibitors selected from the group consisting of 5-[[4-[(2,3-Dimethyl-2H-indazol-6-yl)methylamino]-2 pyrimidinyl]amino]-2-methylbenzolsulfonamide, 5-((S)-6-Methyl-5,6,7,8-tetrahydro-pyrido[3,4-d]pyrimidin-4-yloxy)-indole-1-carboxylic acid [5-(1-methyl-cyclopropyl)-2H-pyrazol-3-yl]-amide; 6-(6-Amino-pyrimidin-4-yloxy)-naphthalene-1-carboxylic acid (3-trifluoromethyl-phenyl)-amide; (-)-5-((S)-7-Acetyl-6-methyl-5,6,7,8-tetrahydro-pyrido[3,4-d]pyrimidin-4-yloxy)-indole-1- carboxylic acid (5-cyclopropyl-isoxazol-3-yl)-amide; 5-(5,6,7,8-Tetrahydro-pyrido[3,4-d]pyrimidin-4-yloxy)-indole-1-carboxylic acid [5-(1-methyl-cyclopropyl)-2H-pyrazol-3-yl]-amide; 1-(2-chloro-4-((6,7-dimethoxyquinolin-4-yl)oxy)phenyl)-3-(5-methylisoxazol-3-yl)urea; and 6-(6-Hydroxymethylpyrimidin-4-yloxy)-naphthalene-1-carboxylic acid (3-trifluoromethyl- phenyl)-amide, or pharmaceutically acceptable salts thereof for use in the manufacture of an oral medicament for dosing to a patient once every 5 to 21 days, once every 5 to 14 days, once every 6 to 10 days, once every 6, 7, or 8 days, or once every 7 days. Said medicament is suitable for use in the treatment of ocular vascular diseases such as age-related macular degeneration, diabetic retinopathy, retinal vein occlusion, and other ophthalmic indications disclosed *infra.*

The medicaments of the invention may also be suitable for use in the treatment of melanoma. More particularly, the medicaments of the invention may be suitable for use in the treatment of ocular melanoma.

### BRIEF DESCRIPTION OF THE DRAWINGS

Figure 1 is a plot of percent inhibition versus oral daily dosage of Compound 2 in the rat CNV model;
Figure 2 is a bar chart of the inhibition of two doses of Compound 2 administered at different dosing intervals to rats in the CNV model of Example 1, 2, and 3;
Figure 3 is a time plot of the concentration of Compound 1 in plasma, retina and posterior eye cup of Brown Norway rats administered a single oral dose of 30 mg/kg at time 0 hours;
Figure 4 is a time plot of the concentration of Compound 2 in plasma, retina and posterior eye cup of Brown Norway rats administered a single oral dose of 10 mg/kg at time 0 hours;
Figure 5 is a time plot of the concentration of Compound 6 in plasma, retina and posterior eye cup of Brown Norway rats administered a single oral dose of 0.3 mg/kg at time 0 hours; and
Figure 6 is a time plot of the concentration of Reference Compound 9 in plasma, retina and posterior eye cup of Brown Norway rats administered a single oral dose of 10 mg/kg at time 0 hours.

### DETAILED DESCRIPTION OF THE INVENTION

The invention is directed to certain dosing regimens for the safe and efficacious treatment of ocular vascular diseases by administration of a VEGF-R2 inhibitor selected from the group consisting of 5-[[4-[(2,3-Dimethyl-2H-indazol-6-yl)methylamino]-2 pyrimidinyl]amino]-2- methylbenzolsulfonamide, 5-((S)-6-Methyl-5,6,7,8-tetrahydropyrido[3,4-d]pyrimidin-4-yloxy)-indole-1-carboxylic acid [5-(1-methyl-cyclopropyl)-2H-pyrazol-3-yl]-amide; 6-(6-Amino-pyrimidin-4-yloxy)-naphthalene-1-carboxylic acid (3-trifluoromethylphenyl)-amide; (-)-5-((S)-7-Acetyl-6-methyl-5,6,7,8-tetrahydro-pyrido[3,4-d]pyrimidin-4-yloxy)-indole-1-carboxylic acid (5-cyclopropyl-isoxazol-3-yl)-amide; 5-(5,6,7,8-Tetrahydropyrido[3,4-d]pyrimidin-4-yloxy)-indole-1-carboxylic acid [5-(1-methyl-cyclopropyl)-2H-pyrazol-3-yl]-amide; 1-(2-chloro-4-((6,7-dimethoxyquinolin-4-yl)oxy)phenyl)-3-(5-methylisoxazol-3-yl)urea; and 6-(6-Hydroxymethyl-pyrimidin-4-yloxy)-naphthalene-1-carboxylic acid (3-trifluoromethyl- phenyl)-amide, or a pharmaceutically acceptable salt thereof, in which said VEGF-R2 inhibitor is administered once every five days or less frequently, once every 5 to 21 days, once every 5 to 14 days, once every 6 to 10 days, or once every 6, 7 or 8 days. Applicants have surprisingly found that said VEGF-R2 inhibitors which exhibit sustained, elevated ocular exposure and rapid systemic clearance are particularly suitable for use in the infrequent dosing regimens of the invention including once a week dosing regimens provided herein. Said VEGF-R2 inhibitors which exhibit selective sustained ocular exposure allow for methods of therapy and medicaments which are infrequently administered.

In one aspect, the invention provides a vascular endothelial growth factor receptor 2 (VEGF-R2) inhibitor or a pharmaceutically acceptable salt thereof for the manufacture of an oral pharmaceutical medicament for use in the treatment an ophthalmic vascular disease, wherein the medicament is for use in dosing regimes in which sequential doses are administered at least 5 days apart and wherein the VEGF-R2 inhibitor is selected from the group consisting of 5-[[4-[(2,3-Dimethyl-2H-indazol-6-yl)methylamino]-2 pyrimidinyl]amino]-2-methylbenzolsulfonamide, 5-((S)-6-Methyl-5,6,7,8-tetrahydro-pyrido[3,4-d]pyrimidin-4-yloxy)-indole-1-carboxylic acid [5-(1-methyl-cyclopropyl)-2H-pyrazol-3-yl]-amide; 6-(6-Amino-pyrimidin-4-yloxy)-naphthalene-1-carboxylic acid (3-trifluoromethyl-phenyl)-amide; (-)-5-((S)-7-Acetyl-6-methyl-5,6,7,8-tetrahydro-pyrido[3,4-d]pyrimidin-4-yloxy)-indole-1-carboxylic acid (5-cyclopropyl-isoxazol-3-yl)-amide; 5-(5,6,7,8-Tetrahydro-pyrido[3,4-d]pyrimidin-4-yloxy)-indole-1-carboxylic acid [5-(1-methyl-cyclopropyl)-2H-pyrazol-3-yl]-amide; 1-(2-chloro-4-((6,7-dimethoxyquinolin-4-yl)oxy)phenyl)-3-(5-methylisoxazol-3-yl)urea; and 6-(6-Hydroxymethyl-pyrimidin-4-yloxy)-naphthalene-1-carboxylic acid (3-trifluoromethyl- phenyl)-amide, or a pharmaceutically acceptable salt thereof.

The use of the first aspect of the invention, the ocular vascular disease is age-related macular degeneration, retinal vein occlusion, diabetic retinopathy, macular edema, or diabetic macular edema.

The use of the first aspect of the invention wherein the sequential doses are administered 6, 7, 8, or 9 days apart. In certain embodiments, the sequential doses are administered 7 days apart.

In certain embodiments, the VEGF-R2 inhibitor for use according to the invention is 5-((S)-6-Methyl-5,6,7,8-tetrahydro-pyrido[3,4-*d*]pyrimidin-4-yloxy)-indole-1-carboxylic acid [5-(1-methyl-cyclopropyl)-2*H*-pyrazol-3-yl]-amide or a pharmaceutically acceptable salt thereof, or the VEGF-R2 inhibitor for use according to the invention is 1-(2-chloro-4-((6,7-dimethoxyquinolin-4-yl)oxy)phenyl)-3-(5-methylisoxazol-3-yl)urea or a pharmaceutically acceptable salt thereof, or the VEGF-R2 inhibitor for use according to the invention is 5-[[4-[(2,3-Dimethyl-2H-indazol-6-yl)methylamino]-2-pyrimidinyl]amino]-2-methylbenzolsulfonamide or a pharmaceutically acceptable salt.

5-((S)-6-Methyl-5,6,7,8-tetrahydro-pyrido[3,4-*d*]pyrimidin-4-yloxy)-indole-1-carboxylic acid [5-(1-methyl-cyclopropyl)-2*H*-pyrazol-3-yl]-amide and 1-(2-chloro-4-((6,7-dimethoxyquinolin-4-yl)oxy)phenyl)-3-(5-methylisoxazol-3-yl)urea, or a pharmaceutically acceptable salt are particularly suitable for use according to the invention.

Periodic oral dosing of VEGF-R2 inhibitors described above wherein the periodicity of dosing once every five days, once every 6 days, weekly dosing or biweekly dosing, provide efficacy against ocular vascular diseases, including age-related macular degeneration, but reduce the systemic, e.g., plasma, exposure of the compound when compared to daily administration. Although not wishing to be bound by theory, the reduced systemic exposure is expected to reduce systemic side effects associated with oral VEGF-R2 inhibitor administration. For example, Compound 2 orally dosed every 6 or 7 days has an improved safety profile compared to Compound 2 administered by daily dosing, dosing every 2 days or dosing every 4 days. See, Examples 6 to 9 *infra.*

The invention also includes the use of VEGF-R2 inhibitor for use according to the invention for treating or preventing ocular vascular disease in a patient comprising administering a weekly dose of said VEGF-R2 inhibitor wherein the weekly dose is between about 0.1 mg and about 800 mg or more preferably 0.5 to 500 mg. The preferred weekly dosage will vary depending on the specific VEGF-R2 inhibitor administered and the size of the patient. In certain embodiments, an efficacious weekly human dose of pazopanib is between about 5 mg and about 800 mg, or preferably between about 30 mg to about 400 mg, and of tivozanib is between about 0.1 mg and about 5 mg, or preferably between about 0.5 mg and about 2 mg. In other embodiments, an efficacious weekly human dose of 5-((*S*)-6-Methyl-5,6,7,8-tetrahydro-pyrido[3,4-*d*]pyrimidin-4-yloxy)-indole-1-carboxylic acid [5-(1-methyl-cyclopropyl)-2*H*-pyrazol-3-yl]-amide or 5-(5,6,7,8-Tetrahydro-pyrido[3,4-*d*]pyrimidin-4-yloxy)-indole-1-carboxylic acid [5-(1-methyl-cyclopropyl)-2*H*-pyrazol-3-yl]-amide is between about 30 mg and about 300 mg or preferably between about 50 mg and about 200 mg. In other embodiments, an efficacious weekly human dose of 6-(6-Amino-pyrimidin-4-yloxy)-naphthalene-1-carboxylic acid (3-trifluoromethyl-phenyl)-amide is between about 5 mg and about 100 mg. In other embodiments, an efficacious weekly human dose of 6-(6-Hydroxymethyl-pyrimidin-4-yloxy)-naphthalene-1-carboxylic acid (3-trifluoromethyl-phenyl)-amide is between about 1 mg and about 50 mg. In other embodiments, an efficacious weekly human dose of (-)-5-((*S*)-7-Acetyl-6-methyl-5,6,7,8-tetrahydro-pyrido[3,4-*d*]pyrimidin-4-yloxy)-indole-1-carboxylic acid (5-cyclopropyl-isoxazol-3-yl)-amide is between 0.1 mg and about 5 mg.

"Ocular vascular disease" as the term is used herein is intended to refer to an ocular disease of the choroid, sclera, retina or related tissue involving abnormal or excessive blood or lymph vessels. Certain ocular vascular diseases which may be treated or prevented include neovascular and dry age-related macular degeneration, geographic atrophy, central serous retinopathy, cystoid macular edema, diabetic retinopathy, proliferative diabetic retinopathy, diabetic macular edema, rubeosis iridis, retinopathy of prematurity, central or branch retinal vein occlusions, inflammatory/infectious retinal neovascularization/edema (e.g. posterior uveitis, sarcoid, toxoplasmosis, histoplasmosis, Vogt-Koyanagi-Harada Disease, multifocal choroiditis, chronic uveitis, tuberculosis, syphilis, punctate and multifocal inner choroidopathy), retinoblastoma, melanoma, ocular tumors, retinal detachment, myopic neovascularization, angioid streaks, Eales disease, Coats disease, Sorsby's fundus dystrophy, ischemic retinopathy (Retinal artery occlusion, Takayasu's, carotid artery occlusion), and choroidal rupture. In certain embodiments, the invention includes the VEGF-R2 inhibitor for use according to the invention in the treatment of wet and dry age-related macular degeneration, diabetic retinopathy, diabetic macular edema, central retinal vein occlusion and branch retinal vein occlusion.

In certain aspects, VEGF-R2 inhibitor for use according to the invention are also suitable for use in the treatment or prevention of melanoma.

As used herein, the term "posterior eye cup" refers to the retinal pigment epithelium, choroid and sclera complex.

As used herein, the term "equivalent dose" refers to a dose in a rat which correlates to a similarly effective dose in a patient or subject, e.g., a similarly effective dose in a human. For example, an equivalent human dose may be correlated between rat and human based on body surface area estimates or other scaling factors. In one non-limiting example a 10 mg/kg orally administered dose in a rate is projected to correlate to a 100 mg dose for a 70 kg human. Final dose correlations between the rat and the subject species (i.e., human) may depend upon a number of biological properties including pharmacokinetic properties, metabolism, bioavailability and the like which are well known in the art.

As used herein, the terms "salt" or "salts" refers to an acid addition or base addition salt of a compound for use according to the invention. "Salts" include in particular "pharmaceutical acceptable salts". The term "pharmaceutically acceptable salts" refers to salts that retain the biological effectiveness and properties of the compounds of this invention and, which typically are not biologically or otherwise undesirable. In many cases, the compounds for use according to the present invention are capable of forming acid and/or base salts by virtue of the presence of amino and/or carboxyl groups or groups similar thereto.

Pharmaceutically acceptable acid addition salts can be formed with inorganic acids and organic acids, e.g., acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, pamoate, phosphate/hydrogen phosphate/dihydrogen phosphate, polygalacturonate, propionate, stearate, succinate, sulfosalicylate, tartrate, tosylate and trifluoroacetate salts.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like.

As used herein, the term "pharmaceutically acceptable carrier" includes any and all solvents, dispersion media, coatings, surfactants, antioxidants, preservatives (*e.g*., antibacterial agents, antifungal agents), isotonic agents, absorption delaying agents, salts, preservatives, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated.

The term "a therapeutically effective amount" of a compound for use according to the present invention refers to an amount of the compound for use according to the present invention that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the compound for use according to the present invention that, when administered to a subject, is effective to (1) at least partially alleviating, inhibiting, preventing and/or ameliorating a condition, or a disorder or a disease (i) mediated by VEGF-R2, or (ii) associated with VEGF-R2 activity, or (iii) characterized by activity (normal or abnormal) of VEGF-R2; or (2) reducing or inhibiting the activity of VEGF-R2; or (3) reducing or inhibiting the expression of VEGF-R2. In another non-limiting embodiment, the term "a therapeutically effective amount" refers to the amount of the compound for use according to the present invention that, when administered to a cell, or a tissue, or a non-cellular biological material, or a medium, is effective to at least partially reducing or inhibiting the activity of VEGF-R2; or at least partially reducing or inhibiting the expression of VEGF-R2. The meaning of the term "a therapeutically effective amount" as illustrated in the above embodiment for VEGF-R2 also applies by the same means to any other relevant proteins/peptides/enzymes, such as VEGF-R2, or other VEGF-R2, and the like.

As used herein, the term "subject" refers to an animal. Typically the animal is a mammal. A subject also refers to for example, primates (*e.g*., humans, male or female), cows, sheep, goats, horses, dogs, cats, rabbits, rats, mice, fish, birds and the like. In certain embodiments, the subject is a primate. In yet other embodiments, the subject is a human.

As used herein, the term "inhibit", "inhibition" or "inhibiting" refers to the reduction or suppression of a given condition, symptom, or disorder, or disease, or a significant decrease in the baseline activity of a biological activity or process.

As used herein, the term "treat", "treating" or "treatment" of any disease or disorder refers in one embodiment, to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (*e.g*., stabilization of a discernible symptom), physiologically, (*e.g*., stabilization of a physical parameter), or both. In yet another embodiment, "treat", "treating" or "treatment" refers to preventing or delaying the onset or development or progression of the disease or disorder.

As used herein, a subject is "in need of" a treatment if such subject would benefit biologically, medically or in quality of life from such treatment.

As used herein, the term "a," "an," "the" and similar terms used in the context of the present invention (especially in the context of the claims) are to be construed to cover both the singular and plural unless otherwise indicated herein or clearly contradicted by the context.

In another aspect, the present invention provides a pharmaceutical composition comprising a compoundfor use according to the present invention and a pharmaceutically acceptable carrier for oral administration. In addition, the pharmaceutical compositions for use according to the present invention can be made up in a solid form (including without limitation capsules, tablets, pills, granules, powders or suppositories), or in a liquid form (including without limitation solutions, suspensions or emulsions). The pharmaceutical compositions can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc.

Typically, the pharmaceutical compositions are tablets or gelatin capsules comprising the active ingredient together with
a) diluents, *e.g*., lactose, dextrose, sucrose, mannitol, sorbitol, cellulose and/or glycine;
b) lubricants, *e.g*., silica, talcum, stearic acid, its magnesium or calcium salt and/or polyethyleneglycol; for tablets also
c) binders, *e.g*., magnesium aluminum silicate, starch paste, gelatin, tragacanth, methylcellulose, sodium carboxymethylcellulose and/or polyvinylpyrrolidone; if desired
d) disintegrants, *e.g*., starches, agar, alginic acid or its sodium salt, or effervescent mixtures; and/or
e) absorbents, colorants, flavors and sweeteners.

Tablets may be either film coated or enteric coated according to methods known in the art.

Suitable compositions for oral administration include an effective amount of a compound for use according to the invention in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets may contain the active ingredient in admixture with nontoxic pharmaceutically acceptable excipients which are suitable for the manufacture of tablets. These excipients are, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

The activity of a compound for use according to the present invention can be assessed by the following *in vitro* & *in vivo* methods.

Following is a description by way of example only.

| Table 1 | |
|---|---|
| Compound # | IUPAC Name |
| 1 | 5-[[4-[(2,3-Dimethyl-2H-indazol-6-yl)methylamino]-2-pyrimidinyl]amino]-2-methylbenzolsulfonamide (Pazopanib) |
| 2 | 5-((*S*)-6-Methyl-5,6,7,8-tetrahydro-pyrido[3,4-*d*]pyrimidin-4-yloxy)-indole-1-carboxylic acid [5-(1-methyl-cyclopropyl)-2*H*-pyrazol-3-yl]-amide |
| 3 | 6-(6-Amino-pyrimidin-4-yloxy)-naphthalene-1-carboxylic acid (3-trifluoromethyl-phenyl)-amide |
| 4 | (-)-5-((*S*)-7-Acetyl-6-methyl-5,6,7,8-tetrahydro-pyrido[3,4-*d*]pyrimidin-4-yloxy)-indole-1-carboxylic acid (5-cyclopropyl-isoxazol-3-yl)-amide |
| 5 | 5-(5,6,7,8-Tetrahydro-pyrido[3,4-*d*]pyrimidin-4-yloxy)-indole-1-carboxylic acid [5-(1-methy)-cyclopropyl)-2*H*-pyrazol-3-yl]-amide |
| 6 | 1-(2-chloro-4-((6,7-dimethoxyquinolin-4-yl)oxy)phenyl)-3-(5-methylisoxazol-3-yl)urea (Tivozanib) |
| 7 | 6-(6-Hydroxymethyl-pyrimidin-4-yloxy)-naphthalene-1-carboxylic acid (3-trifluoromethyl-phenyl)-amide |
| Refence compound 8 | 5-(6,7-dihydro-5H-pyrrolo[3,4-*d*] pyrimidin-4-yloxy)-indole-1-carboxylic acid (5-cyclopropyl-isoxazol-3-yl)-amide |
| Refence compound 9 | 4-[4-[[4-chloro-3-(trifluoromethyl)phenyl]carbamoylamino]phenoxy]-*N*-methyl-pyridine-2-carboxamide (sorafenib) |
| Refence compound 10 | 5-(6-Methylaminomethyl-pyrimidin-4-yloxy)-indole-1-carboxylic acid (1-methyl-5-trifluoromethyl-1*H*-pyrazol-3-yl)-amide |

### Example 1: Rat Laser-Induced Choroidal Neovascularization (CNV) Model; Daily Administration

Three month old Brown Norway rats were administered with placebo or active agent of Table 1 at the dosing concentration and formulation specified in Table 2 *infra* about one hour prior to laser application. The rat eyes were dilated with one drop of phenylephrine and cyclopentolate (*Altaire Pharmaceuticals inc. Aquebogue, NY and Akorn Inc. Lake Forest, IL*) and then immobilized. Each eye was administered four laser burns 2-3 disc diameters from the optic nerve with an Oculight GLx 532 nm laser (Iridex). Each study group had 10 rats, 20 eyes and 80 data points. A successful laser photocoagulation induced a vaporization bubble.

After laser insult, the rats were administered placebo or the compounds of Table 1 at the dosing concentration and formulation specified in Table 2 daily from day 1 to day 11 or 12. Immediately before harvesting the eyes, rodents were injected i.v. with a 2,000 kd fluorescein isothiocyanate (FITC) dextran vascular label. Tissues were harvested about 24 hours after the last dose was administered.

Eyes were fixed with 4% paraformaldehyde and the retinal pigment epithelium(RPE)/choroid/sclera complexes dissected and placed onto microscope slides. Fluorescent images of each CNV lesion were captured and lesion areas were quantified on masked data using Axiovision software (Version 4.5 Zeiss). Inter-group differences were analyzed by one-way analysis of variance (ANOVA) with a Neuman-Keuls post hoc analysis (Prism v. 4.02 by GraphPad Software, Inc. La Jolla CA). Efficacy is defined herein to mean the percentage reduction in CNV area after treatment compared to placebo.

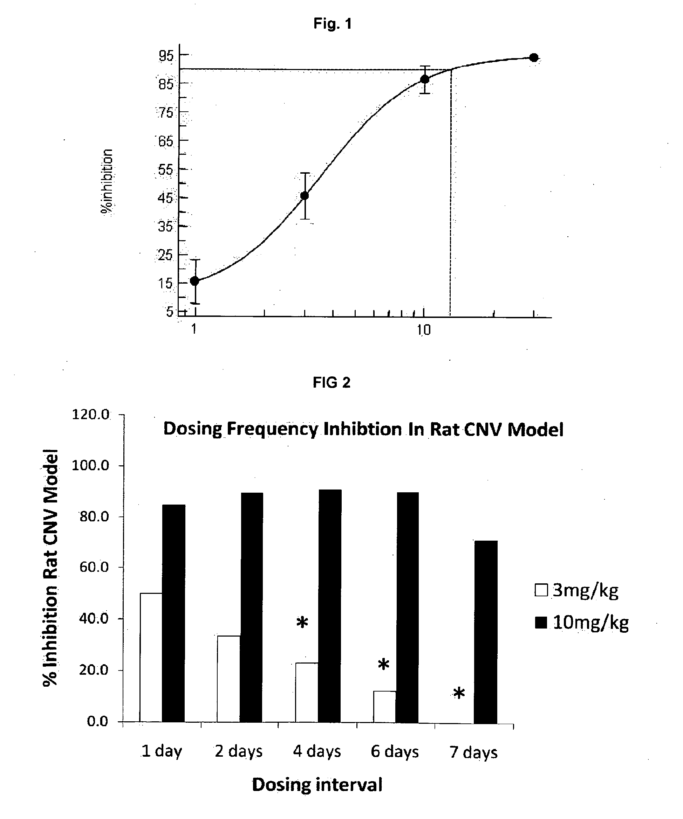

Figure 1 is a plot of the ED₅₀/ED₉₀ plot of daily administration of Compound 2 in the rate model of Example 1. Compound 2 exhibits dose dependent inhibition with daily dosing. In a daily dosing regimen, the ED₅₀ for Compound 2 was 3.3 mg/kg and the ED₉₀ was 13.0 mg/kg. ED₅₀ means that CNV area is 50% smaller than the area of CNV in vehicle treated controls and the ED₉₀ likewise is 90% smaller. N refers to the number of individual rat CNV studies that were completed for a particular dose of Compound 2. Each group consisted of 10 rats, 4 laser burns/eye, and 80 data points/group. Assay length was 11 or 12 days after laser (1 mg/kg n=2, 3 mg/kg n=5, 10 mg/kg n=7, 30 mg/kg n =1).

| Table 2 | | | | |
|---|---|---|---|---|
| Compound # | Formulation | mg/kg | Efficacy Daily % (Ex. 1) | Efficacy Weekly % (Ex. 3) |
| 1 | A | 30 | 42 | 50 |
| 2 | A | 10 | 87 | 71 |
| 3 | A | 1 | 63 | 57 |
| 4 | A | 0.3 | 96 | 50 |
| 5 | A | 10 | 85 | 50 |
| 6 | A | 0.3 | 79 | 40 |
| 6 | A | 1 | 96 | 90 |
| 7 | A | 3 | 96 | 46 |
| Refence compound 8 | A | 3 | 88 | 26 |
| Refence compound 9 | B | 3 | 90 | 26 |
| Refence compound 10 | A | 1 | 88 | 23 |

| | | | | |
|---|---|---|---|---|
| A = 0.5% methyl cellulose, 0.1%, tween 80 in water B = 10% ethanol 90% PEG400 Efficacy is measured as the percent reduction in CNV area in animals treated with compound compared to vehicle control. | | | | |

### Example 2: Rat Laser-Induced Choroidal Neovascularization (CNV) Model; Administration every 2, 4 or 6 days

Three month old Brown Norway rats were administered with placebo or suspension of either 3 or 10 mg/kg of Compound 2 in 0.5% methyl cellulose and 0.1% Tween 80 in water, about one hour prior to laser application. The rat eyes were dilated with one drop of phenylephrine and cyclopentolate (*Altaire Pharmaceuticals inc. Aquebogue, NY and Akorn Inc. Lake Forest, IL*) and then immobilized. Each eye was administered four laser burns 2-3 disc diameters from the optic nerve with a Oculight GLx 532 nm laser (Iridex). A successful laser photocoagulation induced a vaporization bubble. Each study group had 10 rats, 20 eyes and 80 data points.

After the laser insults, the rats were administered placebo or 3 or 10 mg/kg of Compound 2 once every 2 days, every 4 days, or every 6 days. Immediately before harvesting the eyes, rodents were injected i.v. with a 2,000 kd fluorescein isothiocyanate (FITC) dextran vascular label. Tissues were harvested about 24 hours after the last dose was administered. When dosed every two days, the studies were completed on day 11 after laser. When dosed every four or six days, the studies were completed on day 13 after laser

Eyes were fixed with 4% paraformaldehyde and the RPE/choroid/sclera complexes dissected and placed onto microscope slides. Fluorescent images of each CNV lesion were captured and lesion areas were quantified on masked data using Axiovision software (Version 4.5 Zeiss). Inter-group differences were analyzed by one-way analysis of variance (ANOVA) with a Neuman-Keuls post hoc analysis (Prism v. 4.02 by GraphPad Software, Inc. La Jolla CA).

Efficacy is defined herein to mean the percentage reduction in CNV area after treatment compared to placebo.

### Example 3: Rat Laser-Induced Choroidal Neovascularization (CNV) Model; Weekly Administration

Three month old Brown Norway rats were administered with placebo or active agent of Table 1 at the dosing concentration and formulation specified in Table 2 *supra* about one hour prior to laser application. The rat eyes were dilated with one drop of phenylephrine and cyclopentolate (*Altaire Pharmaceuticals inc. Aquebogue, NY and Akorn Inc. Lake Forest, IL*) and then immobilized. Each eye was administered four laser burns 2-3 disc diameters from the optic nerve with an Oculight GLx 532 nm laser (Iridex). Each study group had 10 rats, 20 eyes and 80 data points. A successful laser photocoagulation induced a vaporization bubble.

After laser insults, the rats were administered the compounds of Table 1 formulated as specified in Table 2 or placebo on day 6 and 13. Immediately before harvesting the eyes, rodents were injected i.v. with a 2,000kd Fluorescein isothiocyanate (FITC) dextran vascular label. Tissues were harvested about 24 hours after the last dose is administered.

Eyes were fixed with 4% paraformaldehyde and the RPE/choroid/sclera complexes dissected and placed onto microscope slides. Fluorescent images of each CNV lesion were captured and lesion areas were quantified on masked data using Axiovision software (Version 4.5 Zeiss). Inter-group differences were analyzed by one-way analysis of variance (ANOVA) with a Neuman-Keuls post hoc analysis (Prism v. 4.02 by GraphPad Software, Inc. La Jolla CA). Efficacy is defined herein to mean the percentage reduction in CNV area after treatment compared to placebo.

Figure 2 is a bar chart of the inhibition of two doses of compound 2 administered at different dosing intervals to rats in the CNV model of Example 1, 2, and 3. Statistical confidence of p<0.001 vs. vehicle observed for all 10 mg/kg dosing regimes and 3 mg/kg dosed daily and every 2 days. No statistically significant difference was observed for the 3 mg/kg dose administered every 4, 6, or 7 days compared to vehicle.

Compound 2 administered at 10 mg/kg every 2 days, 4 days, 6 days yielded equivalent efficacy to daily dosing in the rat CNV model of Examples 1 and 2. Weekly administration of Compound 2 provided slightly lower efficacy when compared to daily administration of the 10 mg/kg dose.

Studies were performed to see if less than daily dosing with Compound 2 would provide efficacy. Figure 2 is a bar chart summarizing the efficacy results for the administration of 3 mg/kg or 10 mg/kg of Compound 2 in the rat laser CNV models of Examples 1, 2, and 3. Compound 2 was dosed daily or every 2, 4, 6 and 7 days @ 3 and 10 mg/kg in the rat laser CNV model. In each study, the final dose was given 24 hrs before tissue collection. The study length was from 11 to 14 days in length and specified in examples 1-3. Statistical confidence of p<0.001 vs. vehicle was observed for all 10 mg/kg dosing regimes and 3 mg/kg dosed daily and every 2 days. Statistical confidence of p> 0.05 vs. vehicle was observed for 3 mg/kg dosed every 4, 6 and 7 days.

All compounds tested in Examples 1 and 3 exhibit some efficacy when dosed weekly compared to daily. Compounds 1, 2, 3, and 6 exhibit the greatest level of efficacy at extended time periods between sequential administration of the compound. Reference Compounds 8, 9 and 10 exhibited the least efficacy when administered in a weekly dosing regimen.

### Example 4: Rat Laser-Induced Choroidal Neovascularization (CNV) Model; Administration of a Single Dose

Three month old Brown Norway rats were lasered and not initially treated with placebo or active agent. The rat eyes were dilated with one drop of phenylephrine and cyclopentolate (*Altaire Pharmaceuticals inc. Aquebogue, NY and Akorn Inc. Lake Forest, IL* and then immobilized. Each eye was administered four laser burns 2-3 disc diameters from the optic nerve with an Oculight GLx 532 nm laser (Iridex). Each study group had 10 rats, 20 eyes and 80 data points. A successful laser photocoagulation induced a vaporization bubble.

On either day 13 or day 21 after laser insult, the rats were administered placebo or 10 mg/kg suspension of Compound 2 in 0.5% methyl cellulose and 0.5% and 0.1% Tween 80 in water. Immediately before harvesting the eyes, rodents were injected i.v. with a 2,000 kd fluorescein isothiocyanate (FITC) dextran vascular label. Tissues were harvested about 24 hours after the single dose was administered. I.e. the studies were completed on either day 14 or on day 22 after laser.

Eyes were fixed with 4% paraformaldehyde and the RPE/choroid/sclera complexes dissected and placed onto microscope slides. Fluorescent images of each CNV lesion were captured and lesion areas were quantified on masked data using Axiovision software (Version 4.5 Zeiss). Inter-group differences were analyzed by one-way analysis of variance (ANOVA) with a Neuman-Keuls post hoc analysis (Prism v. 4.02 by GraphPad Software, Inc. La Jolla CA). Efficacy is defined herein to mean the percentage reduction in CNV area after treatment compared to placebo.

Compound 2 or placebo was administered as a single oral dose on either day 13 or day 21 after laser. CNV area measured on day 14 and day 22 in the two experiments. CNV was inhibited by 49% (single dose at day 13) and 43% (single dose at day 21) respectively compared to rats administered placebo. (p < 0.001 vs. vehicle).

### Example 5: Ocular PK in Brown Norway Rats Following Administration of a Single Oral Dose

Three month old Brown Norway rats were administered orally with active agent of Table 1 at the dosing concentration and formulation specified in Table 3 *infra.* Ocular tissues and plasma were collected from 2 rats per active agent at 6, 24, 48, 72, 96, 120 and 144 hrs after dosing. The ocular tissues collected were the retina and the posterior eye cup. Each time point had drug levels measured in 4 individual retinas, 4 individual posterior eye cups and 2 individual plasma samples.

Ocular tissues were homogenized and plasma proteins precipitated and drug concentration was analyzed by LC-MS/MS. Exposures in the Retina, Posterior Eye Cup (PEC) and plasma for Compounds 1, 2, 6 and Reference Compound 9 are listed in Table 4 as area under the curve measurements (AUC).

| Table 3 | | |
|---|---|---|
| Compound | Formulation | Dose |
| Compound 1 | B | 30 mg/kg |
| Compound 2 | A | 10 mg/kg |
| Compound 6 | B | 0.3 mg/kg |
| Reference Compound 9 | A | 10 mg/kg |

| | | |
|---|---|---|
| A = 0.5% methyl cellulose, 0.1%, tween 80 in water B = 10% ethanol 90% PEG400 | | |

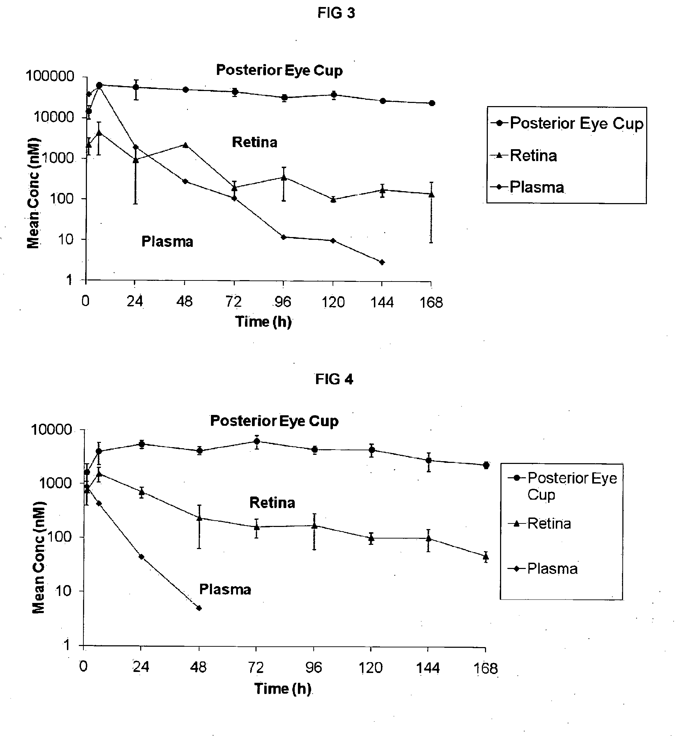

Figure 4 is a plot of concentrations of Compound 2 in the posterior eye cup, retina and plasma. Compound 2 maintained substantially constant concentration in the ocular tissues (e.g., retina and PEC) for 7 days but was cleared relatively quickly from the plasma. Compound 2 has the highest retinal to plasma exposure ratio of these four compounds. The pharmacokinetic profile of Compound 2 provides free drug in the ocular tissues to sustain pharmacological efficacy for at least 7 days. The rapid elimination of Compound 2 from the plasma is expected to minimize undesirable systemic side-effects associated with VEGF-R2 inhibition. Although not wishing to be bound by theory, the bifurcation of systemic (e.g., plasma) exposure from local ocular exposure is believed to provide sustained local efficacy in the treatment of ocular vascular diseases whilst minimizing the risk of on target systemic side effects due to the reduced systemic concentration of Compound 2.

Figure 3 is a plot of concentrations of Compound 1 in the posterior eye cup, retina and plasma. Compound 1 provides sustained ocular exposure in the posterior eye cup tissues for at least a week. However, Compound 1 also exhibits increased plasma exposure throughout the duration of the week after administration.

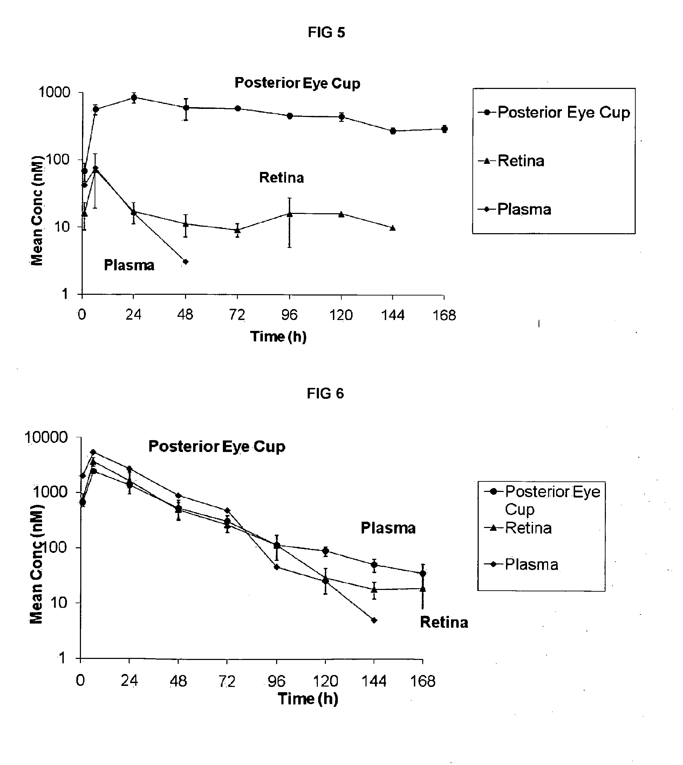

Figure 5 is a plot of concentrations of Compound 6 in the posterior eye cup, retina and plasma. Compound 6 has the ocular profile most similar to Compound 2. However, Compound 6 exhibits a lower retinal to plasma exposure ratio than Compound 2. At 0.3 mg/kg Compound 6 loses 39% efficacy in weekly dosing compared to daily dosing. In contrast, Compound 2 loses 16% efficacy in weekly dosing compared to daily dosing at 10 mg/kg. See, Table 2.

Figure 6 is a plot of concentrations of Reference Compound 9 in the posterior eye cup, retina and plasma. The posterior eye cup, retina and plasma concentration of Reference Compound 9 are substantially similar at each sampling time point. Although not wishing to be bound by theory, the absence of efficacy when Reference Compound 9 is administered in a once a week dosing regimen is believed to be caused by the similar plasma and ocular exposures and the steady decrease in ocular exposure over the course of the week.

| Table 4 | | | | |
|---|---|---|---|---|
| Compound # | AUC₍ₗₐₛₜ₎PEC | AUC₍ₗₐₛₜ₎Retina | AUC₍ₗₐₛₜ₎ Plasma | Retina:plasma |
| 1 | 6819346 | 132036 | 583270 | 0.2 |
| 2 | 726753 | 51865 | 7027 | 7.4 |
| 6 | 83544 | 2471 | 1204 | 0.6 |
| Reference Compound 9 | 82003 | 95132 | 150824 | 2.1 |

### Example 6-8 Histopathological studies in Brown Norway rats orally dosed with Compound 2

Compound 2 or vehicle was orally dosed as a suspension in 0.5% methyl cellulose and 0.1% Tween 80 to Brown Norway rats at doses ranging from of 3 to 30 mg/kg. In one dosing regimen, doses of 3, 10, and 30 mg/kg were given daily for 14 days. Animals in this group did not receive a terminal i.v. injection of Fluorescein isothiocyanate (FITC) dextran vascular label.

In the other dosing regimens, i.e., dosing every 2, 4, or 6 days, rats had laser-induced CNV as described in Example 2. Limited necropsies as well as analysis of CNV were performed on the same individual rats. Starting on day 0 (day of laser application as described earlier) 10 mg/kg of Compound 2 was dosed every 2 days for 11 days, every 4 days for 13 days, or every 6 days for 13 days (summarized in Table 5). Vehicle was dosed on days that Compound 2 was not dosed. At the termination of the study, animals received a 500 µL i.v. injection of FITC-dextran 2,000KD dissolved in PBS (12.5 mg/mL) about 10 minutes before euthanasia. The injection was given to the rats to enable assessment of the area of vasculature of the CNV as described.

Limited necropsy was performed on 5 to 6 rats/ group from each of these studies. The kidneys, heart, brain, gastrointestinal tract (stomach, duodenum, jejunum, ileum, cecum, colon, and rectum), pancreas, and mesenteric lymph nodes were collected from every animal. Tissues were fixed in 10% buffered formalin and routinely processed to microscopic slides. Every tissue was microscopically examined.

| **Table 5** | | | | | | | | | | | | | | | |
|---|---|---|---|---|---|---|---|---|---|---|---|---|---|---|---|
| Dose level (mg/kg) | Day | | | | | | | | | | | | | | |
| | 0 | 1 | 2 | 3 | 4 | 5 | 6 | 7 | 8 | 9 | 10 | 11 | 12 | 13 | 14 |
| 3^{a} | 3 | 3 | 3 | 3 | 3 | 3 | 3 | 3 | 3 | 3 | 3 | 3 | 3 | 3 | Coll |
| 10^{a} | 10 | 10 | 10 | 10 | 10 | 10 | 10 | 10 | 10 | 10 | 10 | 10 | 10 | 10 | Coll |
| 30^{a} | 30 | 30 | 30 | 30 | 30 | 30 | 30 | 30 | 30 | 30 | 30 | 30 | 30 | 30 | Coll |
| 10^{b} | 10 | Veh | 10 | Veh | 10 | Veh | 10 | Veh | 10 | Veh | 10 | Coll | | | |
| 10^{c} | 10 | Veh | Veh | Veh | 10 | Veh | Veh | Veh | 10 | Veh | Veh | Veh | 10 | Coll | |
| 10^{c} | 10 | Veh | Veh | Veh | Veh | Veh | 10 | Veh | Veh | Veh | Veh | Veh | 10 | Coll | |

The number indicates the dose in mg/kg that was given to each group of rats. "Veh" indicates that vehicle was orally administered on that day. "Coll" indicates that the samples were collected from the rats. Superscript (^{a}) refers to Example 6, Superscript (^{b}) refers to Example 7, and Superscript (^{c}) refers to Example 8.

### Example 6 Histopathological findings in Brown Norway rats given Compound 2 daily for 14 days

Brown Norway rats were administered daily 3, 10 or 30 mg/kg doses for 14 days according to the schedule (^{a}) of Table 5. Test article-related changes occurred in the pancreas at doses ≥ 3 mg/kg/day and in kidney and choroid plexus of brain at doses ≥ 10 mg/kg/day. In the kidney, test article-related glomerulopathy was present at doses ≥ 10 mg/kg/day whereas at 30 mg/kg/day, an increased severity of tubular basophilia and inflammatory infiltrates was present. Single cell necrosis of the exocrine pancreas (acinar cells and occasional ductal cells) occurred at doses ≥ 3 mg/kg/day. In the brain, hyalinization/edema of the choroid plexus interstitium and cellular debris affecting small arteries/arterioles were present at doses ≥ 10 mg/kg/day. Additional pancreatic changes included acinar cell atrophy and decreased zymogen granules at doses ≥ 10 mg/kg/day.

### Example 7 Histopathological findings in Brown Norway rats dosed Compound 2 every other 2 days for 11 days

Brown Norway rats were administered daily 10 mg/kg doses for 11 days according to the schedule (^{b}) of Table 5. Test article related changes in the every 2 day dosing group included minimal inflammatory infiltrates that were associated with choroid plexus arterioles. In the kidney minimal to mild glomerulopathy was present. Additionally, an increased severity of tubular basophilia occurred compared to controls, and the incidence of inflammatory cell infiltrates was also increased. In the pancreas, single cell necrosis of pancreatic acinar cells and occasional ductal cells was evident.

### Example 8 Histopathological findings in Brown Norway rats dosed Compound 2 every 4 or 6 days for 13 days

Brown Norway rats were administered daily 10 mg/kg doses for 13 days according to the schedule (^{c}) of Table 5. Test article-related changes in the dosing every 4 day group study included minimal to mild glomerulopathy, minimal choroid plexus changes (cellular debris and/or hyalinization/edema) and/or minimal apoptosis of the exocrine pancreas. Additionally, a single animal in the 10 mg/kg every 4 days group had edema of the large intestine that was characterized by expansion of the lamina propria, diffusely, in a circumferential distribution. Another single animal in the 10 mg/kg every 4 days group had a focal area of hemorrhage and inflammation in the muscularis of the rectum that was considered to be due to the test article.

Under the conditions of the study, animals dosed with 10 mg/kg every 6 days had no test article-related changes.

### Example 9 Histopathological findings in Brown Norway rats dosed Compound 2 weekly for 4 weeks

Compound 2 or vehicle was orally dosed as a suspension in 0.5% methyl cellulose and 0.1% Tween 80 to naive Brown Norway rats at doses ranging from of 3 to 30 mg/kg/week (doses given on days 1, 8, 15, 22 and 29, n= 5 rats/group, details outlined in Table 6).

| Table 6 | | | | | | |
|---|---|---|---|---|---|---|
| Treatment | Day 1 | 8 | 15 | 22 | 29 | 30 |
| Vehicle | X | X | X | X | X | Collect |
| 3 mpk/weekly | X | X | X | X | X | Collect |
| 10 mpk/weekly | X | X | X | X | X | Collect |
| 30 mpk/weekly | X | X | X | X | X | Collect |

| | | | | | | |
|---|---|---|---|---|---|---|
| x indicates day that dose was administered | | | | | | |

At the termination of the study, a complete necropsy was performed and a select list of tissues and organs were examined from control and 30 mg/kg dose groups. Gross lesions and potential target tissues (including kidney, brain, heart and pancreas) were examined from all animals. There were no microscopic test article-related changes present.

## Claims

1. Use of a vascular endothelial growth factor receptor 2 (VEGF-R2) inhibitor or a pharmaceutically acceptable salt thereof for the manufacture of an oral pharmaceutical medicament for use in the treatment an ophthalmic vascular disease, wherein the medicament is for use in dosing regimes in which sequential doses are administered at least 5 days apart and wherein the VEGF-R2 inhibitor is selected from the group consisting of
5-[[4-[(2,3-Dimethyl-2H-indazol-6-yl)methylamino]-2-pyrimidinyl]amino]-2-methylbenzolsulfonamide,
5-((*S*)-6-Methyl-5,6,7,8-tetrahydro-pyrido[3,4-*d*]pyrimidin-4-yloxy)-indole-1-carboxylic acid [5-(1-methyl-cyclopropyl)-2*H*-pyrazol-3-yl]-amide;
6-(6-Amino-pyrimidin-4-yloxy)-naphthalene-1-carboxylic acid (3-trifluoromethyl-phenyl)-amide;
(-)-5-((*S*)-7-Acetyl-6-methyl-5,6,7,8-tetrahydro-pyrido[3,4-*d*]pyrimidin-4-yloxy)-indole-1-carboxylic acid (5-cyclopropyl-isoxazol-3-yl)-amide;
5-(5,6,7,8-Tetrahydro-pyrido[3,4-*d*]pyrimidin-4-yloxy)-indole-1-carboxylic acid [5-(1-methyl-cyclopropyl)-2*H*-pyrazol-3-yl]-amide;
1-(2-chloro-4-((6,7-dimethoxyquinolin-4-yl)oxy)phenyl)-3-(5-methylisoxazol-3-yl)urea; and
6-(6-Hydroxymethyl-pyrimidin-4-yloxy)-naphthalene-1-carboxylic acid (3-trifluoromethylphenyl)-amide, or a pharmaceutically acceptable salt thereof.

2. The use of claim 1, wherein the ophthalmic vascular disease is selected from the group consisting of age-related macular degeneration, diabetic retinopathy, retinal vein occlusion, macular edema or diabetic macular edema.

3. The use of claims 1 or 2, wherein sequential doses are administered 6, 7, 8 or 9 days apart.

4. The use of any one of claims 1 to 3, wherein sequential doses are administered 7 days apart.

5. The use of any one of claims 1 to 4, wherein the VEGF-R2 inhibitor is 5-((*S*)-6-Methyl-5,6,7,8-tetrahydro-pyrido[3,4-*d*]pyrimidin-4-yloxy)-indole-1-carboxylic acid [5-(1-methylcyclopropyl)-2*H*-pyrazol-3-yl]-amide or a pharmaceutically acceptable salt thereof.

6. The use of any one of claims 1 to 4, wherein the VEGF-R2 inhibitor is 1-(2-chloro-4-((6,7-dimethoxyquinolin-4-yl)oxy)phenyl)-3-(5-methylisoxazol-3-yl)urea or a pharmaceutically acceptable salt thereof.

7. The use of any one of claims 1 to 4, wherein the VEGF-R2 inhibitor is 5-[[4-[(2,3-Dimethyl-2H-indazol-6-yl)methylamino]-2-pyrimidinyl]amino]-2-methylbenzolsulfonamide or a pharmaceutically acceptable salt thereof.

## Patentansprüche

1. Verwendung eines Hemmers des Gefäßendothel-Wachstumsfaktor-Rezeptors 2 (VEGF-R2) oder eines pharmazeutisch unbedenklichen Salzes davon zur Herstellung eines oral zu verabreichenden pharmazeutischen Medikaments zur Verwendung zur Behandlung einer Augengefäßkrankheit, wobei das Medikament zur Verwendung in Dosisschemata vorgesehen ist, bei denen sequentielle Dosen mit zumindest 5 Tagen Abstand verabreicht werden, und wobei der VEGF-R2-Hemmer ausgewählt ist aus der Gruppe bestehend aus:
5-[[4-[(2,3-Dimethyl-2H-indazol-6-yl)methylamino]-2-pyrimidinyl]amino]-2-methylbenzolsulfonamid,
5-((*S*)-6-Methyl-5,6,7,8-tetrahydro-pyrido[3,4-*d*]pyrimidin-4-yloxy)-indol-1-carbonsäure-[5-(1-Methyl-cyclopropyl)-2*H*-pyrazol-3-yl]-amid;
6-(6-Amino-pyrimidin-4-yloxy)-naphthalen-1-carbonsäure-(3-trifluormethyl-phenyl)-amid;
(-)-5-((*S*)-7-Acetyl-6-methyl-5,6,7,8-tetrahydro-pyrido[3,4-*d*]pyrimidin-4-yloxy)-indol-1-carbonsäure-(5-cyclopropyl-isoxazol-3-yl)-amid;
5-(5,6,7,8-Tetrahydro-pyrido[3,4-*d*]pyrimidin-4-yloxy)-indol-1-carbonsäure-[5-(1-methyl-cyclopropyl)-2*H*-pyrazol-3-yl]-amid;
1-(2-Chlor-4-((6,7-dimethoxychinolin-4-yl)oxy)phenyl)-3-(5-methylisoxazol-3-yl)harnstoff; und
6-(6-Hydroxymethyl-pyrimidin-4-yloxy)-naphthalen-1-carbonsäure-(3-trifluoromethyl-phenyl)-amid, oder einem pharmazeutisch unbedenklichen Salz davon.

2. Verwendung nach Anspruch 1, wobei die Augengefäßkrankheit aus der Gruppe bestehend aus altersbedingter Makuladegeneration, diabetischer Retinopathie, retinalem Venenverschluss, Makulaödem oder diabetischem Makulaödem ausgewählt ist.

3. Verwendung nach Anspruch 1 oder 2, wobei die sequentiellen Dosen mit einem Abstand von 6, 7, 8 oder 9 Tagen verabreicht werden.

4. Verwendung nach einem der Ansprüche 1 bis 3, wobei die sequentiellen Dosen mit einem Abstand von 7 Tagen verabreicht werden.

5. Verwendung nach einem der Ansprüche 1 bis 4, wobei der VEGF-R2-Hemmer 5-((*S*)-6-Methyl-5,6,7,8-tetrahydro-pyrido[3,4-d]pyrimidin-4-yloxy)-indol-1-carbonsäure-[5-(1-methyl-cyclopropyl)-2*H*-pyrazol-3-yl]-amid oder ein pharmazeutisch unbedenkliches Salz davon ist.

6. Verwendung nach einem der Ansprüche 1 bis 4, wobei der VEGF-R2-Hemmer 1-(2-Chlor-4-((6,7-dimethoxychinolin-4-yl)oxy)phenyl)-3-(5-methylisoxazol-3-yl)harnstoff oder ein pharmazeutisch unbedenkliches Salz davon ist.

7. Verwendung nach einem der Ansprüche 1 bis 4, wobei der VEGF-R2-Hemmer 5-[[4-[(2,3-Dimethyl-2H-indazol-6-yl)methylamino]-2-pyrimidinyl]amino]-2-methylbenzolsulfonamid oder ein pharmazeutisch unbedenkliches Salz davon ist.

## Revendications

1. Utilisation d'un inhibiteur du récepteur 2 du facteur de croissance endothélial vasculaire (VEGF-R2) ou un de ses sels pharmaceutiquement acceptables pour la production d'un médicament pharmaceutique oral destiné à être utilisé dans le traitement d'une maladie vasculaire ophtalmique, le médicament étant destiné à être utilisé en cures de dosage dans lesquelles des doses successives sont administrées à des intervalles d'au moins 5 jours et l'inhibiteur du VEGF-R2 étant choisi dans le groupe constitué par
le 5-[[4-[(2,3-diméthyl-2H-indazol-6-yl)méthylamino]-2-pyrimidinyl]amino]-2-méthylbenzolsulfonamide,
le [5-(1-méthyl-cyclopropyl)-2*H*-pyrazol-3-yl]-amide de l'acide 5-((*S*)-6-méthyl-5,6,7,8-tétrahydro-pyrido[3,4-*d*]pyrimidine-4-yloxy)-indole-1-carboxylique ;
le (3-trifluorométhyl-phényl)-amide de l'acide 6-(6-amino-pyrimidine-4-yloxy)-naphtalène-1-carboxylique ;
le (5-cyclopropyl-isoxazole-3-yl)-amide de l'acide (-)-5-((*S*)-7-acétyl-6-méthyl-5,6,7,8-tétrahydro-pyrido[3,4-*d*]pyrimidine-4-yloxy)-indole-1-carboxylique ;
le [5-(1-méthyl-cyclopropyl)-2*H*-pyrazole-3-yl]-amide de l'acide 5-(5,6,7,8-tétrahydro-pyrido[3,4-*d*]pyrimidine-4-yloxy)-indole-1-carboxylique ;
la 1-(2-chloro-4-((6,7-diméthoxyquinoline-4-yl)oxy)phényl)-3-(5-méthylisoxazole-3-yl)urée ; et
le (3-trifluorométhyl-phényl)-amide de l'acide 6-(6-hydroxyméthyl-pyrimidine-4-yloxy)-naphtalène-1-carboxylique, ou un de leurs sels pharmaceutiquement acceptables.

2. Utilisation selon la revendication 1, dans laquelle la maladie vasculaire ophtalmique est choisie dans le groupe constitué par la dégénérescence maculaire liée à l'âge, la rétinopathie diabétique, l'occlusion veineuse rétinienne, l'oedème maculaire ou l'oedème maculaire diabétique.

3. Utilisation selon les revendications 1 ou 2, dans laquelle les doses successives sont administrées à des intervalles de 6, 7, 8 ou 9 jours.

4. Utilisation selon l'une quelconque des revendications 1 à 3, dans laquelle les doses successives sont administrées à des intervalles de 7 jours.

5. Utilisation selon l'une quelconque des revendications 1 à 4, dans laquelle l'inhibiteur du VEGF-R2 est le [5-(1-méthyl-cyclopropyl)-2*H*-pyrazol-3-yl]-amide de l'acide 5-((*S*)-6-méthyl-5,6,7,8-tétrahydro-pyrido[3,4-*d*]pyrimidine-4-yloxy)-indole-1-carboxylique ou d'un de ses sels pharmaceutiquement acceptables.

6. Utilisation selon l'une quelconque des revendications 1 à 4, dans laquelle l'inhibiteur du VEGF-R2 est la 1-(2-chloro-4-((6,7-diméthoxyquinoline-4-yl)oxy)phényl)-3-(5-méthylisoxazol-3-yl)urée ou un de ses sels pharmaceutiquement acceptables.

7. Utilisation selon l'une quelconque des revendications 1 à 4, dans laquelle l'inhibiteur du VEGF-R2 est le 5-[[4-[(2,3-diméthyl-2H-indazol-6-yl)méthylamino]-2-pyrimidinyl]amino]-2-méthylbenzolsulfonamide ou un de ses sels pharmaceutiquement acceptables.
